# EUROPEAN PATENT SPECIFICATION

(11) **EP 1 900 380 B1**
(45) Date of publication and mention of the grant of the patent: **27.03.2013**
(21) Application number: 06746805.8
(22) Date of filing: 24.05.2006
(51) Int. Cl.: A61K 48/00, A61K 31/7105, A61K 38/00, A61K 47/48, A61L 31/00, A61P 9/00, A61P 9/08, A61K 47/42

(54) **PHARMACEUTICAL COMPOSITION FOR VASCULAR OCCLUSIVE DISEASE**
PHARMAZEUTISCHE ZUSAMMENSETZUNG FÜR OKKLUSIVE GEFÄSSERKRANKUNGEN
COMPOSITION PHARMACEUTIQUE POUR MALADIE OCCLUSIVE VASCULAIRE

(30) Priority: 25.05.2005 JP 2005152346
(43) Date of publication of application: 19.03.2008
(73) Proprietor: CELLMID LIMITED, Sydney NSW 2000 (AU)
(72) Inventor: KADOMATSU, Kenji, National University Corporation, Nagoya-shi, Aichi 4648601 (JP); BANNO, Hiroshi, National University Corporation, Nagoya-shi, Aichi 4648601 (JP); TAKEI, Yoshifumi, National University Corporation, Nagoya-shi, Aichi 4648601 (JP); KOMORI, Kimihiro, National University Corporation, Nagoya-shi, Aichi 4648601 (JP); MURAMATSU, T., National University Corporation, Nagoya-shi, Aichi 4648601 (JP); SAKUMA, Sadatoshi, Cell Signals Inc., Yokohama-shi, Kanagawa 2300046 (JP)
(74) Representative: Woods, Geoffrey Corlett
(86) International application number: PCT/JP2006/310375
(87) International publication number: WO 2006/126600

(56) References cited:
- WO-A1-00/10608
- WO-A1-01/97857
- WO-A1-03/000297
- WO-A2-2004/033620
- JP-A- 2004 275 169
- HONMA K ET AL: "The role of atelocollagen-based cell transfection array in high-throughput screening of gene functions and in drug discovery" CURRENT DRUG DISCOVERY TECHNOLOGIES, BENTHAM SCIENCE PUBLISHERS, NL, vol. 1, no. 4, 1 December 2004 (2004-12-01), pages 287-294, XP008092990 ISSN: 1570-1638
- HAYASHI K. ET AL.: 'Antisense oligodeoxyribonucleotide as to the growth factor midkine suppresses neointima formation induced by balloon injury' AM. J. PHYSIOL. HEART CIRC. PHYSIOL. vol. 288, no. 5, 06 January 2005, page H2203-9, XP003004185
- MINAKUCH Y. ET AL.: 'Atelocollagen-mediated synthetic small interfering RNA delivery for effective gene silencing in vitro and in vivo' NUCLEIC ACIDS RES vol. 32, no. 13, 2004, page E109, XP003004186

## Description

### [Technical Field]

Present invention relates to a pharmaceutical composition for preventing or treating an obstructive vascular disease including vascular restenosis after angioplasty.

### [Background of Art]

Cardiac and vascular pathologies have kept on increasing in recent years. Amongst these, angiostenotic pathologies caused by, for instance, arteriosclerosis of coronary arteries or the like, have also been on the increase. For instance, percutaneous transluminal coronary angioplasty (PTCA), which was incepted in 1977 for angiostenotic pathologies of coronary arteries, has become ever since a widespread revascularization method. Percutaneous transluminal coronary angioplasty is a method for enlarging blood vessels and regenerating blood flow by expanding a balloon attached to the tip of a catheter, in a stenotic site. However, 30 to 60% of patients exhibit restenosis at the regenerated site within 6 months. This has detracted from the efficacy of percutaneous transluminal coronary angioplasty. Further combining PTCA with stent placement, in which a stent is placed with a view to keeping the blood vessel expanded at the stenotic site, allows reducing restenosis frequency, but the restenosis rate in the stent placement site is still of about 15 to 20%, while in some cases the restenosis rate exceeds 30 to 60% in patients having multiple diseases. Surgical vein grafting, which is performed for occlusive diseases in peripheral arteries, suffers also from a restenosis rate of about 20%.

Suppression of angiostenotic pathologies has also been essayed through antisense oligonucleotides using liposomes for expressing Midkine (MK), which is a growth /differentiation factor (Hayashi et al., Am J Physiol Heart Circ Physiol. 2005 May;288(5):H2203-9. Epub 2005 Jan 6.). There have been also attempts at applying antisense oligonucleotides, using liposomes, in canine graft models (Matsumoto T, Komori K, Yonemitsu Y, Morishita R, Sueishi K, Kaneda Y, Sugimachi K. Hemagglutinating virus of Japan-liposome-mediated gene transfer of endothelial cell nitric oxide synthase inhibits intimal hyperplasia of canine vein grafts under conditions of poor runoff. J Vasc Surg. 1998; 27: 135-144:article 49). There are also current developments in the application of various antisense oligonucleotides and/or aspirin in various animals such as rabbits and the like employing pluronic gel (from BASF), which gels at a temperature close to the body temperatures of mammals Fulton GJ, Davies MG, Koch WJ, Dalen H, Svendsen E, Hagen PO. Antisense oligonucleotide to proto-oncogene c-myb inhibits the formation of intimal hyperplasia in experimental vein grafts. J Vasc Surg. 1997; 25: 453-463, Fulton GJ, Davies MG, Barber L, Svendsen E, Hagen PO. Locally applied antisense oligonucleotide to proliferating cell nuclear antigen inhibits intimal hypertrophy in experimental vein grafts. Ann Vasc Surg. 1998; 12: 412-417, Suggs WD, Olson SC, Madnani D, Patel S, Veith FJ. Antisense oligonucleotides to c-fos and c-jun inhibit intimal hypertrophy in a rat vein graft model. Surgery, 1999; 126: 443-449, Torsney E, Mayr U, Zou Y, Thompson WD, Hu Y, Xu Q. Thrombosis and neointima formation in vein grafts are inhibited by locally applied aspirin through endothelial protection. Circ Res. 2004; 94: 1466-1473: articles 14,15, 23, 24). Application of antisense oligonucleotides in graft models, using adenoviruses, has also been reported (Yamashita A, Hanna AK, Hirata S, Dardik A, Sumpio BE. Antisense basic fibroblast growth factor alters the time course of mitogen-activated protein kinase in arterialized vein graft remodeling. J Vasc Surg. 2003; 37: 866-873).

### [Disclosure of the Invention]

Such vascular restenosis appears to be caused by intimal hypertrophy derived from proliferation and migration of vascular smooth muscle cells that result from injury-induced inflammation of the inner wall, in particular the endothelium, of blood vessels during the above-described surgical procedures. However, the above sustained drug release stents are problematic in that, on account of the nonspecific cytotoxicity of the drug, regeneration of endothelial cells is particularly difficult, which hinders reversion to a normal vascular wall, and are also problematic in that, although effective against restenosis of coronary arteries in the heart, their effect against peripheral artery stenosis remains inconclusive. These problems are yet to be solved. Also, inhibition of MK by antisense oligonucleotides (inhibition to about 60% of the control) was arguably insufficient. The other methods described above, moreover, afforded at best only inhibition up to about 60% of the control. Therefore, there is still demand for superior prevention or therapy of obstructive vascular diseases such as vascular restenosis or the like.

As a result of research on the use of a nucleic acid construct for inhibiting expression of MK protein through RNA interference, in the presence of a collagen molecule, the inventors perfected the present invention upon discovering that combining such a nucleic acid construct with a collagen molecule resulted in an inhibitory effect unexpectedly superior to the conventionally discovered inhibitory effect of intimal hypertrophy exerted by MK antisense oligonucleotides. The present invention provides the following means.

The present invention provides a pharmaceutical composition for use in the treatment or prevention of obstructive vascular disease which results from restenosis after vascular reconstructive surgery comprising;
a nucleic acid construct inhibiting expression of Midkine gene through RNA interference wherein said nucleic acid construct comprises siRNA which targets at least a portion of mRNA coding for Midkine protein, and
a collagen molecule, wherein the administration site is the outer layer of the blood vessel of the obstructive vascular disease site.

The nucleic acid construct comprises siRNA. The collagen molecule may be an atelocollagen molecule. The collagen molecule may comprise a fibrous collagen molecule. Such a composition for obstructive vascular diseases can be in any one form selected from powder, fibers, liquid, gel, pellet, film, sponge and tube. Such a composition for obstructive vascular diseases can be used for prevention or treatment of occlusive diseases of blood vessels, such as coronary arteries in the heart, cerebral blood vessels, renal blood vessels and peripheral blood vessels resulting from restenosis after vascular reconstructive surgery. Such a composition may be used for prevention or treatment of obstructive vascular disease which results from restenosis after vascular reconstructive surgery in humans. The nucleic acid construct may target any one sequence selected from sequences as set forth in SEQ ID Nos. 11 to 14.

The present invention provides a medicinal device for treatment or prevention of obstructive vascular disease comprising a support for vessel treatment, and any one of the above-described pharmaceutical compositions for obstructive vascular disease, carried on at least part of the surface of the support to be enabled to gelate at the obstructive vascular disease site wherein said pharmaceutical composition is provided at a site abutting the outer layer of the blood vessel wall. The support for vessel treatment may be a stent.

### [Brief Description of the Drawings]

Fig. 1 shows the sequences of siRNA target sites in rMK mRNA.
Fig. 2 shows the structure of each siRNA.
Fig. 3 is a photograph showing Western blotting for the expression level of rMK protein in RK13 cells.
Fig. 4 is a graph showing densitometry quantification of the intensity of band detected by Western blotting shown in Fig. 3.
Fig. 5 is a photograph showing the time course of Western blotting for the expression of rMK protein in the control graft.
Fig. 6 is a photograph showing the effect on rMK-expressing RK13 cells originating from rabbit kidney (the expression level of rMK 24 hours after transfection).
Fig. 7 is a graph showing densitometry quantification for the intensity ratio of bands of rMK protein and β-actin in Western blotting on postoperative day 7 of the graft treated by siRNA #2-ST and control graft.
Fig. 8 is a photograph showing a result of immunohistochemical observation on postoperative day 14 of a graft removed from the control graft model.
Fig. 9 shows the result of confocal laser microscopy observation on postoperative day 7 of frozen sections of a graft removed from the graft model introduced with siRNA #2-ST.
Fig. 10 shows tissue samples of graft model introduced with siRNA #2-ST (a) and control graft model (b) 4 weeks after surgery.
Fig. 11 is a graph comparing the thickness of tunica intima between the vessels shown Fig. 10(a) and (b).
Fig. 12 is a graph comparing the ratio of the thickness of tunica media to that of tunica intima between the vessels shown in Fig. 10(a) and (b).
Fig. 13 is a diagram showing an example of structure of each siRNA that suppresses the expression of human MK through RNA interference.

### [Best Mode For Carrying Out The Invention]

The pharmaceutical composition for obstructive vascular disease of the present application comprises a nucleic acid construct inhibiting expression of the Midkine gene through RNA interference, and a collagen molecule. Also, the medicinal device for obstructive vascular disease of the present invention comprises a support for vessel treatment, and the pharmaceutical composition carried on at least part of the surface of the support for vessel treatment.

The pharmaceutical composition for obstructive vascular disease and the medicinal device for obstructive vascular disease comprise both a nucleic acid construct inhibiting expression of the Midkine gene through RNA interference, and a collagen molecule. By combining thus the nucleic acid construct and the collagen molecule, the nucleic acid construct effectively suppresses expression of MK protein and effectively suppresses injury-induced intimal hypertrophy. Although collagen molecules have been conventionally used as supports for various medicines, the advantages of using a combination of a collagen molecule and a nucleic acid construct aimed at RNA interference of MK, for the treatment of obstructive vascular diseases, have not been hinted at heretofore. The affinity of collagen molecules for vascular tissue, the stable conservation and sustained-releasability of the nucleic acid construct afforded by the collagen molecules, together with the MK expression inhibition effected by the nucleic acid construct, allow obtaining an intimal hypertrophy inhibitory effect that, although not meant to constrain the present invention in any way, arguably exceeds expectations. Presumably, the remarkable inhibition of intimal hypertrophy is afforded thanks to the combination of the above nucleic acid construct and a collagen molecule, whereby the above nucleic acid construct is kept in the vicinity of the affected site by a collagen matrix, over a certain lapse of time at a relatively early stage after injury of vascular endothelium, thus effectively inhibiting expression of Midkine at an early stage after injury. Specifically, the intimal hypertrophy inhibition effect that is obtained beyond expectations, and which results from the possibility of inhibiting Midkine expression at an appropriate timing for suppressing intimal hypertrophy, is presumably afforded by combining the nucleic acid construct and the collagen molecule.

That is because expression of MK in human adults is fundamentally very limited and weak, so that it is hardly conceivable that inhibition of MK protein expression in endothelial cells leads to cellular death. The invention holds promise of being effective not only against restenosis of coronary arteries in the heart, but also against peripheral artery stenosis.

Preferred embodiments of the pharmaceutical composition and medical device for obstructive vascular disease of the present invention are explained next. The medical composition and the medical device of the present invention can be used in humans and in non-human mammals.

### (Pharmaceutical composition for obstructive vascular disease)

### (Nucleic acid construct)

The nucleic acid construct of the present invention is constructed so as to inhibit expression of the Midkine gene by RNA interference. In the present specification, the term "nucleic acid" means a polynucleotide such as deoxyribonucleic acid and ribonucleric acid. This term encompasses single-stranded (sense or antisense) or double-stranded polynucleotides, wherein the polynucleotides may be modified naturally or artificially. In the present invention, "RNA interference" refers to the phenomenon whereby double-stranded RNA sequence-specifically degrades mRNA of a target gene. RNA interference allows thus inhibiting the expression of a target gene. Herein, inhibition of the expression of the Midkine gene means inhibition of the translation into a polypeptide by mRNA coding for Midkine, or reduction in the amount of expression of the protein Midkine.

Midkine (MK), which is a growth /differentiation factor that was discovered as a gene product transiently expressed in the differentiation induction process mediated by retinoic acid in embryonic carcinoma cells (EC), is a 13 kDa polypeptide rich in basic amino acids and cysteine. (Kadomatsu, K. et al.: Biochem. Biophys. Res. Commun., 151: 1312-1318; Tomomura, M. et al.: J. Biol. Chem., 265:10765-10770, 1990). MK, which is found from humans to Xenopus, exhibits high interspecies conservation, with the amino acid sequence having 87% homology in humans and mice. MK possesses varied bioactivity. Specifically, MK promotes neuritic extension, neuron survival, and is believed to participate in the construction of the nervous system. MK has been found also in injured tissues, many human cancers and in Alzheimer's senile plaques, having attracted attention also as regards its role in tissue repair, fibrinolytic system activation power and disease condition. Since expression of MK increases in many human cancers (Aridome, K. et al.: Jap. J. Cancer Res., 86: 655-661, 1995), and since cells resulting from transfection and expression of MK cDNA exhibit malignant transformation (Kadomatsu, K., et al.: Brit. J. Cancer, 75: 354-359,1997), MK is hypothesized to be intimately involved in the genesis and progression of human cancers. In knockout mice lacking the MK gene, neointimal formation decreases dramatically in balloon injury models, which suggests the involvement of MK also in the occurrence of vascular pathologies (Horiba, M. et al.: J. Clin. Invest., 105: 489-495, 2000).

The nucleic acid construct, which is constructed to enable inhibition of the Midkine gene, has, as a target thereof, at least a portion of mRNA coding for Midkine protein. One form of such a nucleic acid construct includes a RNA construct having a mutually hybridizing oligoribonucleotide of double-stranded structure. Specifically, the nucleic acid construct may comprise a relatively short double-stranded oligoribonucleotide (small interference RNA: siRNA) having protruding 3' termini, or a single oligoribonucleotide (short hairpin RNA = shRNA) forming (or having) a hairpin structure.

siRNA, which has a sense sequence and an antisense sequence corresponding to a target sequence, has formed therein a double-stranded structure in which the sense sequence and the antisense sequence are hybridized over a certain length. The sense sequence and the antisense sequence hybridize with each other, but may partially possess unpaired portions. For instance, the sense sequence may have one or more mismatched bases and/or deleted bases. Although the sense sequence and antisense sequence of siRNA have protruding 3' termini and a double-stranded portion for pairing, over predefined respective lengths, identity of the sense sequence or complementarity of the antisense sequence vis-à-vis the target sequence of the mRNA on the overhanging 3' termini are not necessarily required.

shRNA has a sense sequence on the 5' side, and an antisense sequence on the 3' side, corresponding to the same target sequence of siRNA. These sequences form a stem over a given length and have a loop site in between that has a nuclease processing site. Thus, shRNA yields siRNA through intracellular processing. As already explained for siRNA, the sense sequence and antisense sequence corresponding to overhangs of the siRNA derived from shRNA need not have identity and complementarity vis-à-vis the target sequence.

Typically, the double-strand length of the sense sequence and the antisense sequence in such an embodiment of the nucleic acid construct ranges preferably from 13 to 28 bp, more preferably from 13 to 27 bp, yet more preferably from 19 to 21 bp, and is most preferably of 19 or 20 bp. Typically, the sense sequence and antisense sequence comprising a 3' side structure not forming a double strand has preferably 15 to 30 nt, more preferably 15 to 29 nt, yet more preferably 21 to 23 nt, and most preferably 21 or 22 nt. The protruding 3' terminus of the siRNA comprises, preferably, 2 to 4 nt, and more preferably 2 nt. The length of the loop of the shRNA may be such that it does not hinder forming and maintaining a double strand (stem of the shRNA) and a 3' terminus structure.

The mRNA sequence of the MK targeted by such a nucleic acid construct is determined, for instance, in accordance with the rules below, which allow designing a suitable siRNA.
(1) Targeting sequences that yield, for instance, AA(N19)TT, AA(N21), NA(N21) in the CDS of the target gene (using 19 bases of the 3^{rd} to 21^{st} base, as siRNA),
(2) Leaving at least 50 to 100 bases downstream from a start codon, to avoid binding sites of transcription factors,
(3) Avoiding the vicinity of 5'-UTR,
(4) Selecting sequences having no homology with other genes, through homology search,
(5) Setting a GC content of about 50%.

siRNA design methods including methods for determining other target sequence can be suitably carried out inaccordance with the rules disclosed in, for instance, http://design.rnai.jp/sidirect/index.php, http://www.rockefeller.edu/labheads/tuschl/sirna.html and Rational siRNA design for RNA interference (Nature Biotechnology, vol. 22, 326- 330 (2004), Angela Reynolds, Devin Leake, Queta Boese, Stephen Scaringe, William S Marshall & Anastasia Khvorova), Improved and automated prediction of effective siRNA (Biochem Biophys Res Commun. 2004 Jun 18;319(1):264-74, Chalk AM, Wahlestedt C, Sonnhammer EL.).

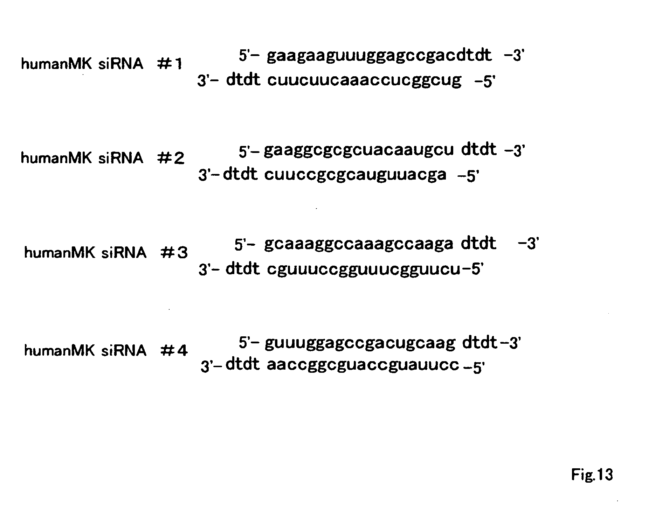

For instance, mRNA of human MK has already been reported (Tsutsui, J. et al, Biochem. Biophys. Res. Commun., 176:792-797, 1990, GenBank accession number: NM_001012334). On the basis of such sequences there can be designed and constructed a nucleic acid construct such as siRNA or the like that allows inhibiting expression of mRNA of human MK. For instance, the target sites 1 through 4 disclosed in Japanese Unexamined Patent Application Laid-open No. 2004-275169 may be used as the target sequence. Fig. 13 illustrates an example of human MK siRNA that can be used as a nucleic acid construct capable of expressing RNA interference for these target sequences. In MK siRNA, each of sense sequences and antisense sequences can have 2nt overhang portion at its the end. The nucleic acid construct such as siRNA or the like capable of inhibiting MK expression allows effectively preventing and treating obstructive vascular diseases in humans. A preferred target site is target site 4.

mRNA target site 1: gaaggaguuuggagccgac; GC CONTENT 52%)(SEQ ID: 11)
mRNA target site 2: gaaggcgcgcuacaaugcu; GC CONTENT 52%)(SEQ ID: 12)
mRNA target site 3: gcaaaggccaaagccaaga; GC CONTENT 47%)(SEQ ID: 13)
mRNA target site 4: guuuggagccgacugcaag; GC CONTENT 52%)(SEQ ID: 14)

Such a nucleic acid construct can be synthesized in accordance with known polyribonucleotide chemical synthesis methods such as the phosphoamidite method. The nucleic acid construct can also be synthesized using in vitro transcription. In vitro transcription can be carried out, for instance, by synthesizing DNA coding for such polyribonucleotides; on the basis of this DNA, synthesizing a double-stranded DNA template for transcription by PCR using DNA polymerase and a primer having a RNA promoter sequence such as T7RNA promoter or the like; and employing then RNA polymerase for the double-stranded DNA template for transcription. This allows obtaining a desired single-stranded RNA. In the case of siRNA, this can be obtained by manufacturing a double-stranded RNA through hybridization of the obtained antisense RNA and sense RNA, by degrading appropriate termini using RNases or the like, and by purifying the obtained double-stranded RNA. In the case of shRNA, the latter can be obtained by manufacturing single-stranded RNA comprising a sense sequence, an antisense sequence and a loop sequence, trimmed using various base-specific RNases, between the sense sequence and the antisense sequence, followed by annealing of the sense sequence and the antisense sequence. siRNA can be obtained by treating with base-specific nucleases the loop sequence of the shRNA thus obtained. The in vitro transcription method is not limited to the foregoing. In vitro transcription can be carried out in accordance with various known methods, employing commercially available in vitro transcription kits. The nucleic acid construct may also comprise various known modifying groups for nuclease resistance.

Another form of the nucleic acid construct is a vector coding for the RNA construct of the above form, i.e., a vector coding expressibly for siRNA and/or shRNA. In such a form, a shRNA expression vector can be constituted by an antisense sequence, a sense sequence and, besides, a loop sequence, in such a way that single-stranded RNA to which shRNA is constructibly connected is expressed by intracellular transcription. A siRNA expression vector may be constructed to afford transcription of RNA having a predefined sense sequence and antisense sequence. In a siRNA expression vector, the sense sequence and the antisense sequence may be expressed by a single vector, or by respective different vectors.

Promoters used for such expression vectors may be pol II or pol III promoters, provided that they allow production of corresponding RNA by the above DNA. Pol III promoters include, for instance, U6 promoter, tRNA promoter, retroviral LTR promoter, adenovirus VA1 promoter, 5S rRNA promoter, 7SK RNA promoter, 7SL RNA promoter, H1 RNA promoter and the like. Pol II promoters include, for instance, cytomegalovirus promoter, T7 promoter, T3 promoter, SP6 promoter, RSV promoter, EF-1α promoter, β-actin promoter, γ-globulin promoter, SRα promoter and the like.

The expression vector may be in the form of a plasmid vector or a virus vector. The type of vector is not particularly restricted, and may be selected in accordance with, for instance, the cell to be transfected. Vectors for mammalian cells include, for instance, virus vectors such as retrovirus vectors, adenovirus vectors, adeno-associated virus vectors, vaccinia virus vectors, lentivirus vectors, herpes virus vectors, alphavirus vectors, EB virus vectors, papillomavirus vectors, foamy virus vectors and the like.

The nucleic acid construct using such expression vectors can be easily constructed, for instance, on the basis of commercially available vectors constructed for siRNA and/or shRNA manufacture, or on the basis of protocols of such vectors, and/or on the basis of "RNAi Experiment Protocols, Experimental Medicine, Supplementary Volume" (Yodosha, published October 1, 2004).

### (Collagen molecule)

The pharmaceutical composition of the present invention contains a collagen molecule. As the collagen molecule there may be used soluble collagen or solubilized collagen. "Soluble collagen" includes, for instance, collagen that is soluble in acidic or neutral water, or in salt-containing water. "Solubilized collagen" includes, for instance, enzyme-solubilized collagen solubilized by enzymes and alkali-solubilized collagen solubilized by an alkali.

The source of collagen is not particularly restricted, and there may be used collagen extracted from vertebrates or genetic recombinants thereof. Herein there can be used, for instance, collagen extracted from mammals, birds, fishes or genetic recombinants thereof. The collagen type, which ranges from I to V, is not particularly limited, and any type may be used. Examples of the collagen type include type I collagen, obtained through acid extraction of mammalian dermis or a genetic recombinant thereof. More desirably, the collagen type may be, for instance, type I collagen obtained by acid extraction from calf dermis or type I collagen produced by genetic engineering. Collagen derived from calf dermis or human dermis is used preferably as the type I collagen produced by genetic engineering. Atelocollagen from which high-antigenicity telopeptides are enzymatically removed, or atelocollagen produced by genetic engineering, are desirable in terms of safety. Herein, atelocollagen having three or less tyrosine residues per molecule is yet more preferable.

Preferably, the collagen molecule of the pharmaceutical composition fibrates at least prior to administration of the pharmaceutical composition into the body, or immediately upon administration at a predetermined administration site, under the physiological conditions of that site, or quickly thereafter (at most, within several tens of minutes, preferably within several minutes) (hereinafter, such timings after administration are collectively referred to as "upon administration"). Fibration of collagen molecules takes place through assembly of collagen molecules along the longitudinal axis of the molecules, depending on temperature, pH and/or salt concentration. Fibration of the collagen molecule is believed to increase the stability of the nucleic acid construct inside the body. Through fibration of the collagen molecule at the administration site, the fibrous collagen molecule becomes stably supported at the administration site, i.e. at the inner wall and/or outer wall of a blood vessel, or at a medical material for intravascular placement.

The pharmaceutical composition, containing a collagen molecule that fibrates upon administration, contains the nucleic acid construct and the collagen molecule prior to fibration. The pharmaceutical composition may also be mixed, immediately prior to administration, with a liquid agent that confers fibration ability to the collagen molecule, so as to effect collagen molecule fibration. The pharmaceutical composition may also be such that fibration takes place under physiological conditions at the administration site without using such a liquid agent. The pharmaceutical composition may comprise the nucleic acid construct and an already-fibrous collagen molecule. In that case, the collagen molecule undergoes fibration preferably by mixing homogeneously beforehand, prior to collagen molecule fibration, the nucleic acid construct and the collagen molecule. As the case may require, the collagen molecule after fibration may take any of the various forms described below, such as powder, pellet, film or sponge, through drying or the like.

The fibrous collagen molecule may be suitably crosslinked. The presence of a crosslinked structure is believed to delay degradation in the body and to delay the release of the nucleic acid construct. Collagen can be imparted a crosslinked structure by means of various known methods, such as irradiation, for instance UV irradiation, or transglutaminase treatment, provided that the effectiveness of the pharmaceutical composition is preserved. Succinyl groups may also be introduced in the fibrous or non-fibrous collagen molecule, with a view to conferring antithrombogenicity thereto.

The present composition may be formulated in any form including, for instance, powder, fibers, liquid, film, sponge, tube and the like. Such formulations may be arrived at as a result of the form of the collagen molecules themselves, or by using additives and/or excipients conventionally known in the technical field in question.

When in the form of a solution to gel, the pharmaceutical composition ranges from about 0. 001% to about 10%, preferably from about 0.1 % to about 5%, and more preferably from about 1.5% to about 3.75%. When in the form of a powder, fibers, pellet, film, sponge or tube, the pharmaceutical composition may range from about 5% to about 99%, preferably from about 10% to about 70%, and more preferably from out 25% to 50%. The pharmaceutical composition may comprise a salt or the like that contributes to create the conditions for collagen molecule fibration.

The pharmaceutical composition is used locally in a form that is appropriate for the formulation thereof on a site where a thrombotic-occlusive disease has occurred or a site where a thrombotic-occlusive disease can occur (such a site is called hereinafter, for instance, an obstructive vascular disease site). In addition to an actual obstructive site occurring in a blood vessel, the term obstructive vascular disease site denotes the whole blood vessel of the obstructive site, including the outer-layer of the blood vessel wall at the obstructive site. The pharmaceutical composition in a solution or gel form can be injected into, or applied onto, the outer layer of a blood vessel wall of an obstructive vascular disease site; the pharmaceutical composition in powder or fiber form can be spread on the outer wall of an obstructive vascular disease site; while the pharmaceutical composition in the form of a tube, pellet, film or sponge may be placed and/or adhered against the inner or outer layer of a blood vessel wall of an obstructive vascular disease site. The pharmaceutical composition can be supported easily at an obstructive vascular disease site by, for instance, making the pharmaceutical composition into a tube, film or sponge that can conform or adapt to the inner or outer layer of a blood vessel wall obstructive vascular disease site in an obstructive vascular disease site. For local application of the pharmaceutical composition of the present invention there may be used a percutaneous procedure involving injection of the pharmaceutical composition employing a catheter, balloon or the like, or a surgical procedure such as microsurgery.

The pharmaceutical composition can be made to gel easily at an obstructive vascular disease site, upon administration, when the collagen molecule of the pharmaceutical composition is contained therein in such a way so as to fibrate readily upon administration, or is fibrated beforehand. As a result, this allows keeping the nucleic acid construct, which is the active component against intimal hypertrophy, stably localized and at the intended concentration.

As explained above, using the pharmaceutical composition containing the above nucleic acid construct and collagen molecules in an obstructive vascular disease site allows achieving an intimal hypertrophy inhibition effect beyond expectations. In particular, the pharmaceutical composition can be preferably used for prevention or treatment of vascular occlusive diseases, such as coronary arteries in the heart, cerebral blood vessels, renal blood vessels and peripheral blood vessels, which result from, for instance, restenosis after vascular reconstructive surgery or arteriosclerosis.

### (Medicinal device for obstructive vascular disease)

The medicinal device for obstructive vascular disease of the present invention comprises a support for vessel treatment, and the pharmaceutical composition carried on at least part of the surface of the support for vessel treatment. Such a medicinal device for an obstructive vascular disease allows stably supplying the pharmaceutical composition to an obstructive vascular disease site. When using an intravascular-insertable support for vessel treatment, the pharmaceutical composition can be supplied at an obstructive vascular disease site or the like percutaneously, without resorting to a surgical procedure.

The support for vessel treatment includes vascular reconstruction materials such as materials that reinforce, function as a prosthesis, support and/or replace at least part of a blood vessel in the body, and includes also implements for intravascular insertion. Specific examples of vascular reconstruction materials include, for instance, artificial blood vessels and vascular prostheses, while intravascular insertion implements include, for instance, intravascular placement implements such as stents or the like, as well as catheters and balloons. Using a vascular reconstruction material or an intravascular placement implement, such as a stent or the like, enables long-term indwelling of the pharmaceutical composition at an obstructive vascular disease site, and allows preventing also vascular occlusions such as restenosis or the like due to lesions incurred during placement of the foregoing. When a catheter or a balloon is used, the pharmaceutical composition can be easily supplied also to a peripheral blood vessel, where placement of an indwelling implement is difficult. A catheter or a balloon may also be used for supplying the pharmaceutical composition to an indwelling implement such as a stent, and/or to a vascular reconstruction material. The forms and materials of such implements for intravascular insertion may be those used in conventional implements for intravascular insertion.

A stent can be used as a support for vessel treatment, as it allows preventing effectively restenosis that accompanies stent placement and the like. The stent used may have a coil form, a tubular web form or the like, and may be a balloon-expandable stent or a self-expandable stent.

The pharmaceutical composition is provided on at least part of the surface of the support for vessel treatment. A pharmaceutical composition can be provided at a site corresponding to the inner wall of a blood vessel or the outer wall of the blood vessel, or at a site abutting these sites. When the support for vessel treatment is a biograft, an artificial blood vessel or a vascular prosthesis, a pharmaceutical composition may be provided on the blood-vessel inner wall or the blood-vessel outer wall of the foregoing. In the case of an intravascular placement implement such as a stent or the like, or in the case of a catheter or balloon, a pharmaceutical composition may be provided at a site (elongation portion, expansion portion or the like) that can abut the inner wall of the blood vessel. According to the present invention, the administration site is the outer layer of the blood vessel of the obstructive vascular disease site.

Prior to use, the medicinal device is in a state where the pharmaceutical composition is supported, in the form of powder, fibers, gel, film, sponge or the like, on the surface of the support. The method by which the pharmaceutical composition is supported on the support surface is not particularly limited. The pharmaceutical composition in solution form or gel form may be soaked into, or applied onto, the support for vessel treatment, followed by gelling or drying as-is, thereby becoming supported on the support for vessel treatment. If necessary, collagen fibration may also be carried out. Moreover, the pharmaceutical composition in the form of, for instance, gel, film or sponge, conforming to the outer-face shape or inner-face shape of the support for vessel treatment, may be closely adhered to the support for vessel treatment via a suitable adhesive layer interposed optionally therebetween. In that case, the collagen molecule may be crosslinked.

The pharmaceutical composition is supplied surgically or percutaneously to an obstructive vascular disease site in accordance with the form of the support for vessel treatment. Since it contains collagen molecules, the pharmaceutical composition on the medicinal device becomes supported on the surface of the support for vessel treatment in a gelled state (preferably, through collagen molecule fibration) resulting from absorption of water inside the body brought about when the pharmaceutical composition reaches obstructive vascular disease site, or at most within several tens of minutes thereafter.

Since, as explained above, the pharmaceutical composition is supported on the support for vessel treatment, the medicinal device allows the pharmaceutical composition to exert its effect stably at the placement site of the support for vessel treatment. Therefore, the medicinal device has the pharmaceutical composition provided on the surface of the support for vessel treatment, which is apt to cause vascular restenosis, and hence the medicinal device allows realizing simultaneously vascular reconstruction and restenosis prevention. In particular, the pharmaceutical composition can be preferably used for prevention or treatment of vascular occlusive diseases, such as coronary arteries in the heart, cerebral blood vessels, renal blood vessels and peripheral blood vessels, which result from, for instance, restenosis after vascular reconstructive surgery or arteriosclerosis.

Described herein is a method for preventing or treating a obstructive vascular disease using the above-described pharmaceutical composition or medicinal device.

### [Example 1]

In the present Example, five types of siRNAs were prepared based on the genetic sequence of rabbit MK (hereinafter referred to as rMK; GenBank Accession No. AY553907) and their knockdown effects were assessed in vitro.

### <Preparation of siRNA>

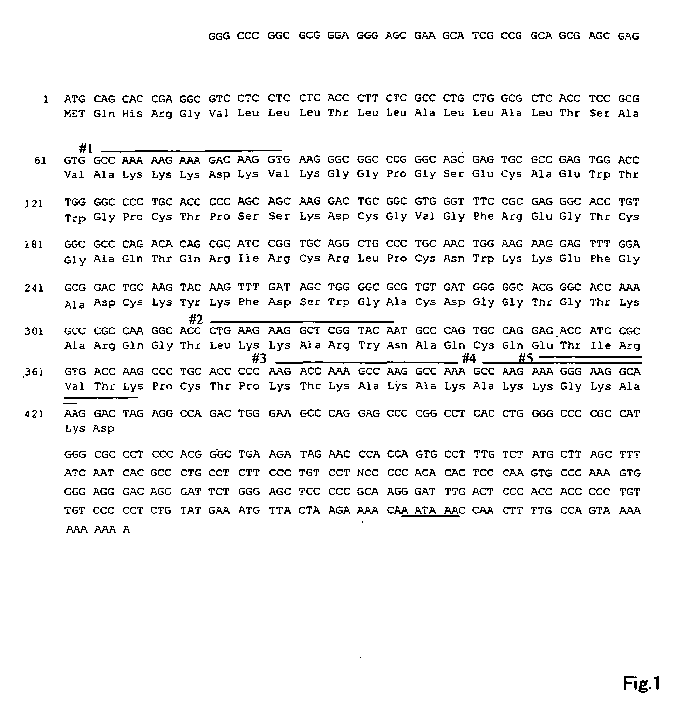

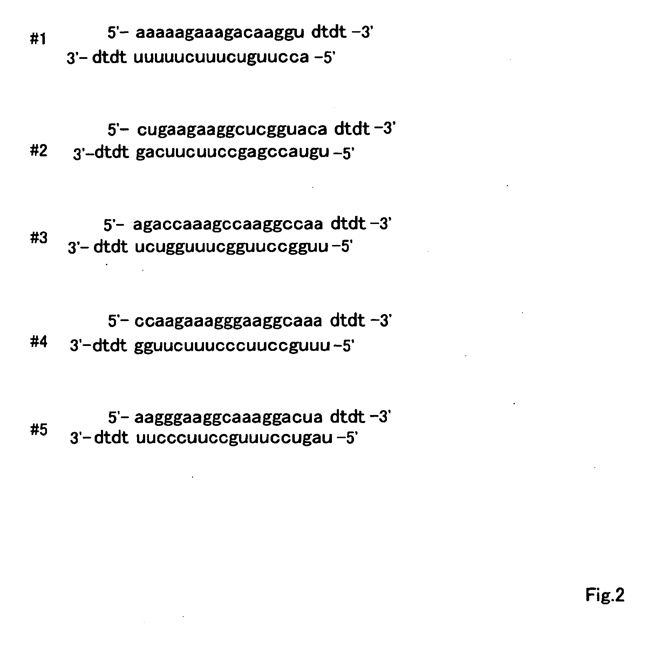

Fig. 1 shows the sites targeted in the mRNA of rMK by the five types of siRNAs (#1 to #5) that were prepared. The sequence in Fig. 1 is in a form that includes the CDS (DNA). The sense sequences of the synthesized siRNAs are shown respectively in SEQ ID NOs: 1 to 5, and Fig. 2 shows the structures of each of the synthesized siRNAs.

### (Evaluation of siRNA)

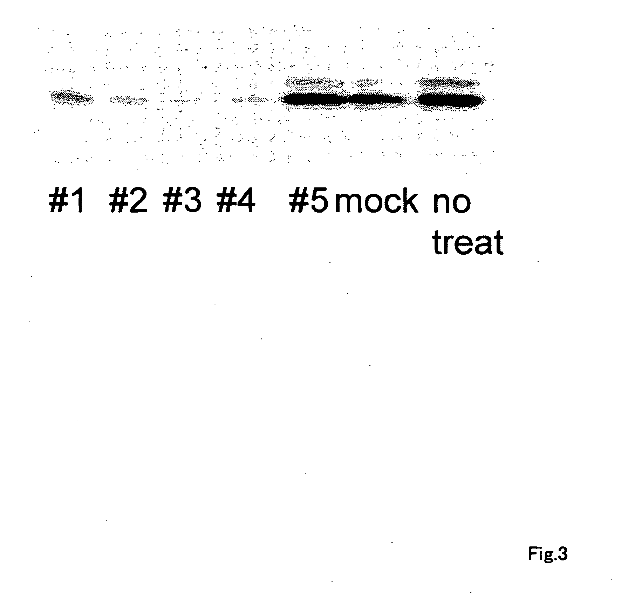

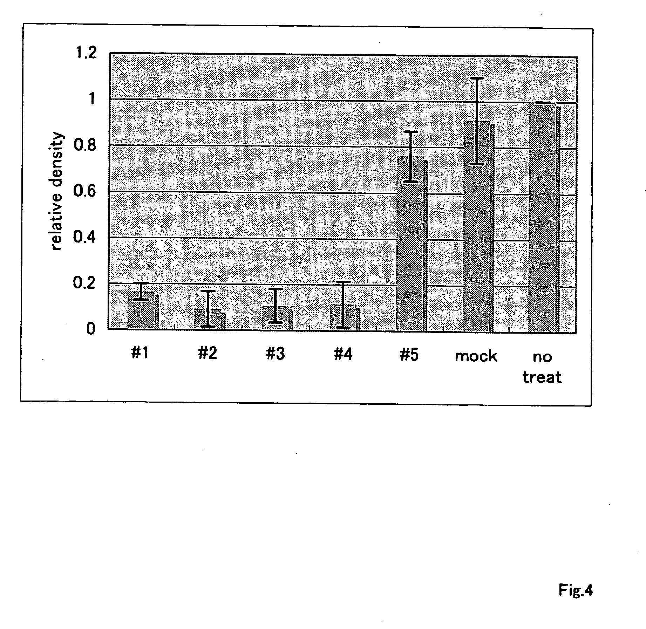

The day before siRNA transfection, MK-expressing RK13 cells derived from rabbit kidney cells were plated onto a 3-cm plate and was preincubated until just under 50% confluency. Each of the mixed solutions individually containing 5 *µ* L of 20 *µ* M solutions of the five prepared siRNA and 95 *µ* L of optiMEM were mixed in advance and this was combined with a solution mixture containing 3 mL of LipofectAMINE2000 and 95 mL of optiMEM that had been incubated at RT for 5 minutes. The resulting solution mixture was incubated for 20 minutes at room temperature, and was added to an RK13 cell culture dish in which the medium had been exchanged in advance with 800 mL of 10% FCS/DMEM. After 48 hours, the medium was exchanged to heparin-containing DMEM (serum-free) and the culture supernatant was collected 24 hours later. The collected culture supernatant was subjected to SDS gel electrophoresis (SDS-PAGE) (15% gel) and then Western blotting using an anti-MK antibody was performed according to the method of Muramatsu et al. (Muramatsu, H. et al.: Dev. Biol. 159:392-402, 1993). Fig. 3 shows the results of Western blotting, and Fig. 4 shows a graph produced by digitizing the concentration of the bands detected by Western blotting using densitometer analysis. "Mock" had the same composition as the solution mixture mentioned above except that it did not contain siRNA.

As shown in Figs. 3 and 4, #1 is approximately 18%, #2 is approximately 8%, and #3 and #4 were approximately 10% respectively, when the untreated band is defined as 100%. Therefore, siRNA#2, the siRNA with the highest suppressive effect, was selected. Furthermore, the antisense strand of siRNA#2 was taken as a scramble sequence, and a double-stranded RNA complementary to this sequence was synthesized (scramble siRNA; hereinafter, simply referred to as siRNA-SCR). A chemical modification was performed on these two types of double stranded RNAs, siRNA#2 and siRNA-SCR, using siSTABLE™ (Dharmacon; for in vitro studies) to synthesize, siRNA#2 siSTABLE (hereinafter, referred to as siRNA#2-ST) and siRNA-SCR siSTABLE (hereinafter, referred to as scramble siRNA-SCR-ST).

### [Example 2]

Whether siRNAs targeting MK will prevent occlusion of a venous graft was determined in the present Example.

### (Preparation of a venous graft model)

Rabbit external jugular vein was collected under general anesthesia, and common carotid interposition bypass surgery was performed. Then, a low-blood-flow model was prepared by ligating all peripheral blood vessels (including internal jugular vein) except for the first branch of the external jugular vein. Treatments such as this have been reported to enhance intimal hypertrophy by decreasing peripheral vascular bed and lowering the blood flow (Am. J. Physiol. Heart Circ. Physiol. 2004; 286:240-245).

### (siRNA solution)

12 mL of 500 *µ*M solutions of each of siRNA#2, siRNA-SCR, siRNA#2-ST, and siRNA-SCR-ST prepared in Example 1, and 12 mL of physiological saline solution were mixed with an equal amount of 3.5% atelocollagen solution (KOKEN). The final collagen concentration was 1.75% and the siRNA concentration was 10 *µ* M. Each of these solutions were applied to the total circumference of the venous graft after anastomosis in the prepared venous graft model, and after waiting for gelation to take place, the incision was closed. Cy3-labeled siRNA#2-ST was used to examine the efficiency of introduction into the walls of the venous graft.

### <Evaluation>

After a predefined number of days had passed after the operation, venous grafts were collected from each type of venous graft model, and the following items were examined.

### (Expression of rMK protein)

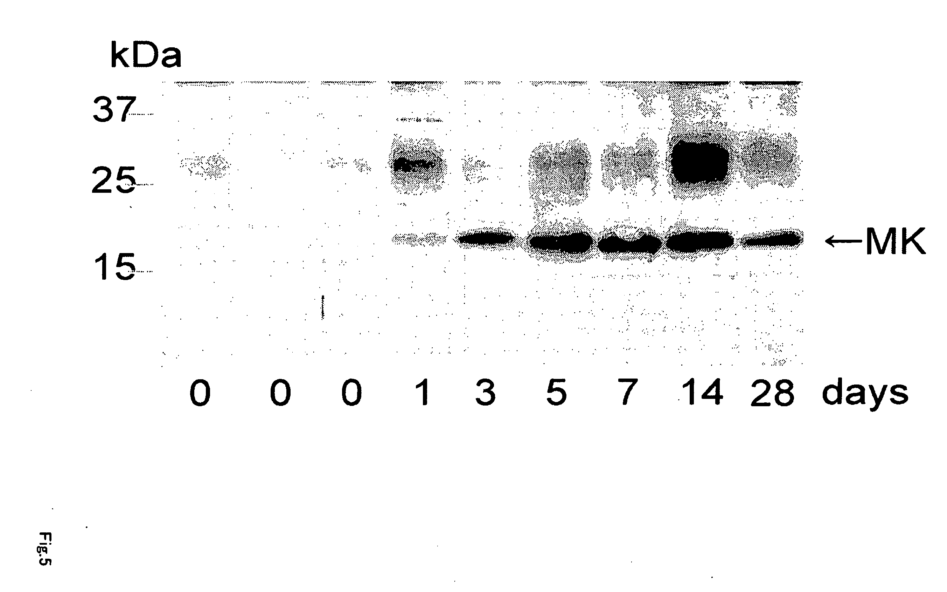

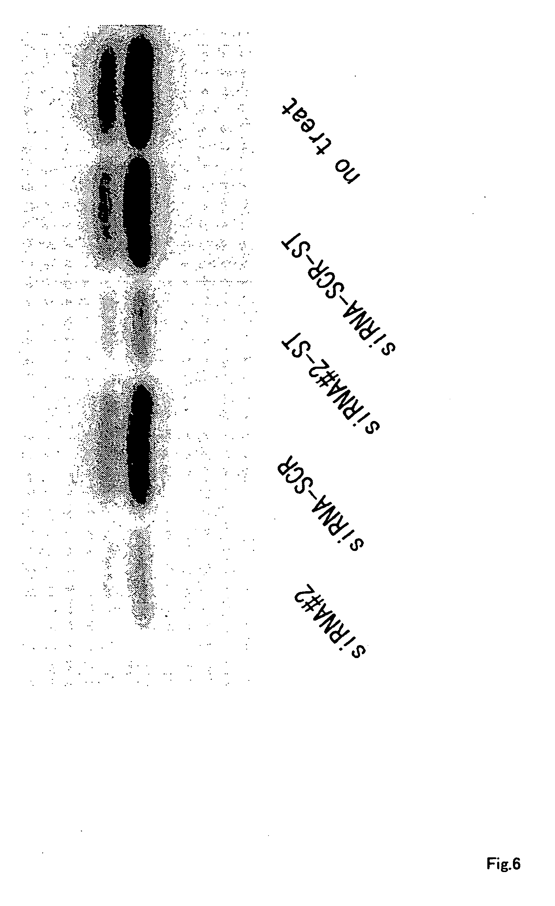

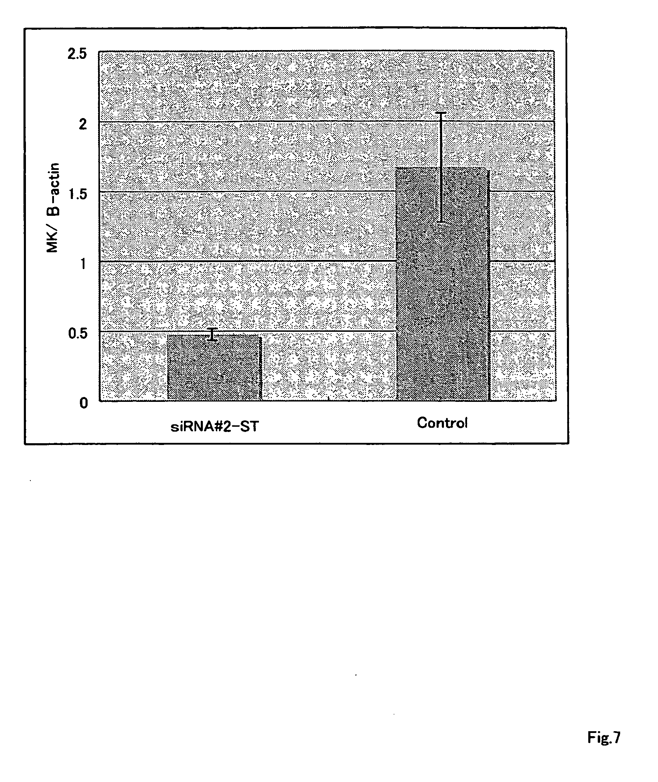

The expression of rMK protein was evaluated by homogenizing all tissues of the graft removed from the venous graft model, and then subjecting this to Western blotting (primary antibody: goat anti-human MK, and secondary antibody: rabbit anti-goat IgG). Expression of rMK protein was also compared to the level of beta-actin expression. Fig. 5 shows the change in rMK protein expression over time using a Western blot of a control graft to which an atelocollagen solution that does not contain siRNA and only contains physiological saline solution was applied; Fig. 6 shows effects on rabbit kidney-derived RK13 cells that express rMK (rMK expression level 24 hours after transfection); and Fig. 7 shows a graph produced by digitizing, using densitometer analysis, the concentration ratio of the rMK protein and beta-actin protein bands in a Western blot of a graft on which siRNA#2-ST was used and a control graft, 7 days after operation.

### (Localization of rMK protein)

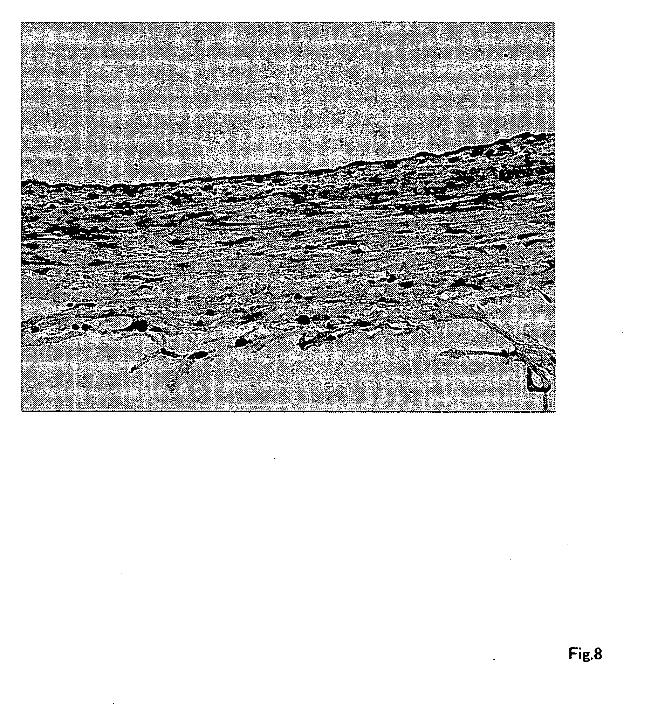

Localization of the rMK protein was evaluated by immunohistological staining of paraffin sections of a graft removed from a venous graft model using chicken anti-mouse MK as the primary antibody and biotin-labeled rabbit anti-chicken IgG as the secondary antibody. The results of observations made on a graft removed from the control graft model 14 days after operation are shown in Fig. 8.

### (Localization of siRNA)

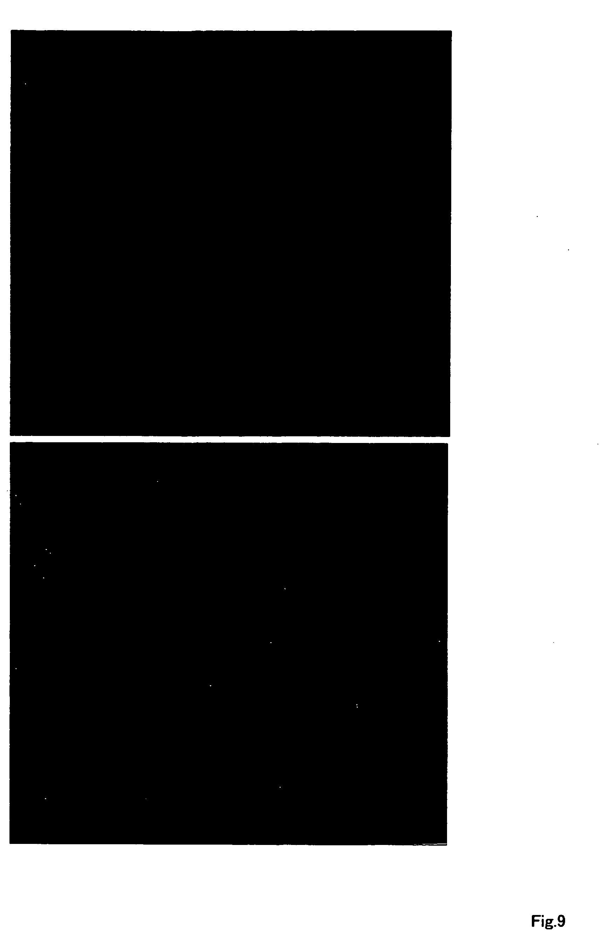

Frozen sections of a graft removed from the Cy3-labeled siRNA#2-ST-introduced graft model were examined directly using a confocal laser microscope. Confocal laser micrographs of frozen sections of a graft removed from a model 7 days after operation are shown in Fig. 9.

### (Morphological analysis of tissue samples)

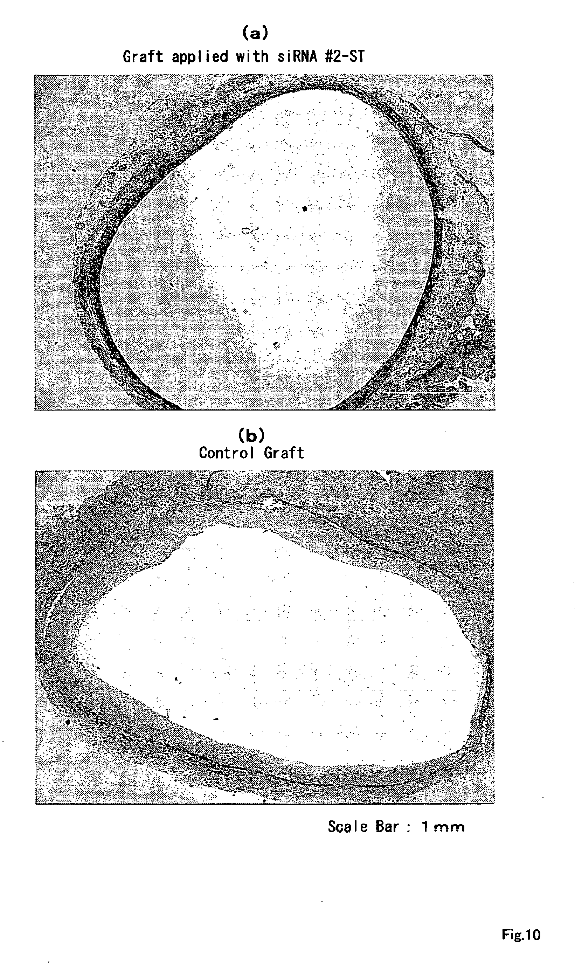

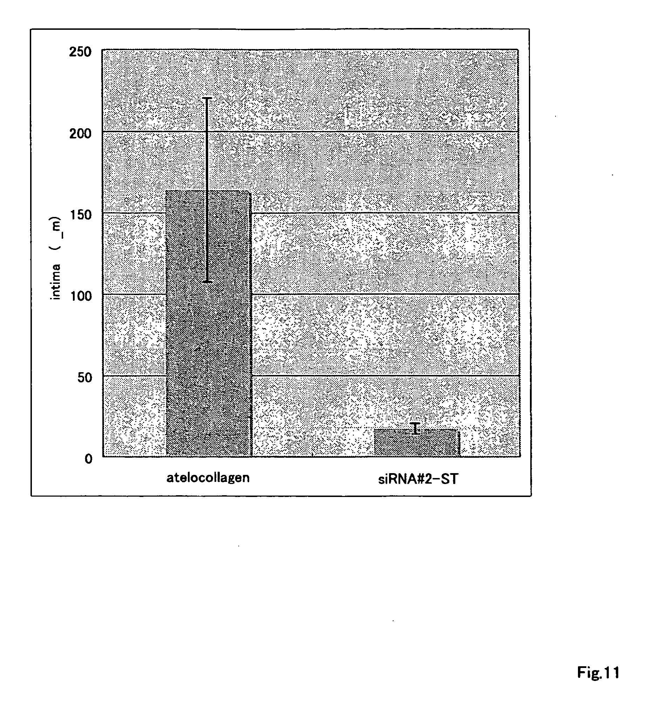

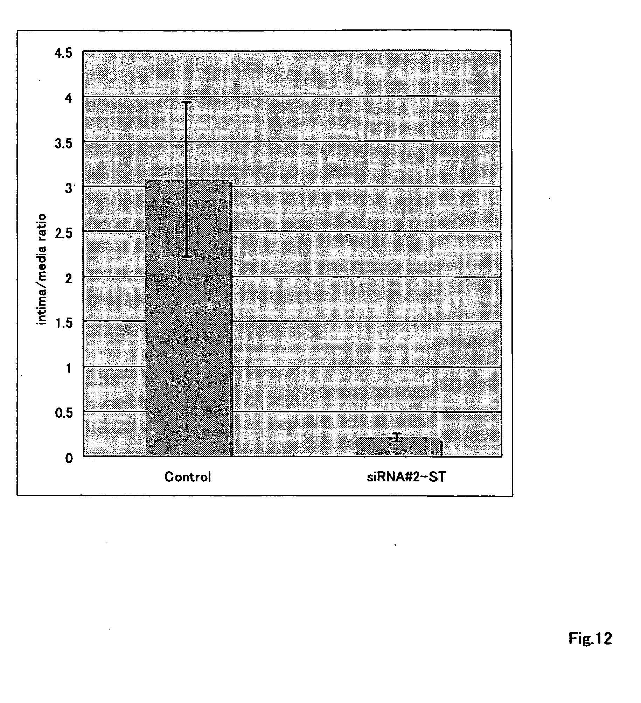

Paraffin sections of each of the removed grafts were Elastica van Gieson stained. Tissue samples of the siRNA#2-ST-introduced graft model and the control graft model four weeks after operation are shown in Fig. 10, and comparative diagrams of the thickness of vascular intima and ratio of the thickness of the vascular intima with respect to the vascular media are shown in Figs. 11 and 12, respectively.

Judging from the time course of rMK protein expression in the control graft model shown in Fig. 5, rMK expression starts few days after operation and peaks at 7 to 14 days after operation. According to the images observed by immunohistological staining shown in Fig. 8, strong rMK expression (dark color) is observed in the neointima 14 days after operation, and this supported the results shown in Fig. 5.

Western blotting of rMK protein in the various siRNA-introduced graft models shown in Fig. 6 showed that MK siRNA#2 and siRNA #2-ST individually suppress rMK protein expression specifically and at the same level. Furthermore, according to the graph shown in Fig. 7 comparing the band densities of rMK protein against beta-actin in the control model and in the siRNA #2-ST-introduced model seven days after operation, rMK expression in the siRNA #2-ST-introduced graft was suppressed to approximately one fourth that of the control graft. More specifically, rMK expression was found to be effectively suppressed at 7 days after operation when rMK expression normally peaks.

Furthermore, according to confocal laser micrographs of grafts on which Cy3-labeled siRNA #2-ST had been used, which are shown in Fig. 9, Cy3 labels were observed in all of the intramural layers of the graft seven days after operation, and this showed that siRNA was introduced into the cells of all of the intramural layers of the graft.

According to the tissue samples of the graft to which siRNA #2-ST had been applied and the control graft four weeks after operation, which are shown in Fig. 10, the vascular intimal hypertrophy was found to be significantly suppressed in the graft to which siRNA #2-ST had been applied. As shown in Figs. 11 and 12, based on these tissue samples, in the graft to which RNA #2-ST was applied, the ratio of vascular intimal thickness/vascular medial thickness was 6% or so of the control graft, and the thickness of the intima itself was found to be suppressed to 10% or so of the control graft.

The above revealed that by administering siRNA against MK with a collagen molecule, the siRNA is retained at the administered site, reaches the vascular intima or adjacent sites, and suppresses MK protein expression. It was also found that such treatment can effectively suppress thickening of the vascular intima.

### [Industrial Applicability]

The present invention is available for treating or preventing an obstructive vascular disease.

### [Sequence Listing Free Text]

SEQ ID NOs.1 to 5: Target sequences of siRNAs to rabbit midkine
SEQ ID NOs.6 to 10: Sense chains of anti-rMK siRNAs
SEQ, NOs.11 to 14: Target sequences of SiRNAs to human midkine
SEQ ID NOs. 15 to 19: Antisense chains of anti-rMK siRNAs
SEQ ID NOs.20 to 27: Sequences of anti-human MK siRNAs
SEQ ID NO. 28: Rabbit midkine DNA sequence
SEQ ID NO. 29: Rabbit midkine protein sequence
SEQ ID NOs. 30 to 32: Sequences targeted in the CDS of the target MK gene

## Claims

1. A pharmaceutical composition for use in the treatment or prevention of obstructive vascular disease which results from restenosis after vascular reconstructive surgery comprising;
a nucleic acid construct inhibiting expression of Midkine gene through RNA interference wherein said nucleic acid construct comprises siRNA which targets at least a portion of mRNA coding for Midkine protein, and
a collagen molecule, wherein the administration site is the outer layer of the blood vessel of the obstructive vascular disease site.

2. The pharmaceutical composition according to claim 1, wherein said collagen molecule comprises atelo-collagen molecule.

3. The pharmaceutical composition according to any one of claims 1 to 2, wherein said collagen molecule contains a fibrous collagen molecule.

4. The pharmaceutical composition according to any one of claims 1 to 3, wherein said use in the treatment or prevention of obstructive vascular disease comprises the inhibition of intimal hypertrophy.

5. The pharmaceutical composition according to any one of claims 1 to 4, wherein said composition is administered through gelation of said composition in the outer layer of the blood vessel wall of the obstructive vascular disease.

6. The pharmaceutical composition according to any one of claims 1 to 5, wherein said composition is used for prevention or treatment of post-PTCA restenosis or occlusions of vessels including coronary artery, cerebral vascular, renovascular and peripheral vessel.

7. The pharmaceutical composition according to any one of claims 1 to 6, wherein said nucleic acid constructs targets any one sequence of sequences as set forth in SEQ ID NOs.11 to 14.

8. A medicinal device for the treatment or prevention of obstructive vascular disease, comprising:
a support for vessel treatment,
pharmaceutical composition according to any one of claims 1 to 7, carried on at least a part of the surface of the support to be enabled to gelate at the obstructive vascular disease site, wherein said pharmaceutical composition is provided at a site abutting the outer layer of the blood vessel wall.

9. The medicinal device according to claim 8, wherein said medical device is an artificial blood vessel or a vascular prosthesis.

## Patentansprüche

1. Pharmazeutische Zusammensetzung zur Verwendung bei der Behandlung oder Prävention obstruktiver Gefäßerkrankung, welche durch Restenose nach rekonstruktiver Gefäßchirurgie entsteht, umfassend
ein Nukleinsäure-Konstrukt, welches Expression des Midkin-Gens durch RNA-Interferenz inhibiert, wobei besagtes Nukleinsäure-Konstrukt siRNA umfasst, welche zumindest einen Teil der Midkin-Protein-kodierenden mRNA targetiert, und
ein Kollagenmolekül, wobei die Verabreichungsstelle die äußere Schicht des Blutgefäßes der obstruktiven Gefäßerkrankungsstelle ist.

2. Pharmazeutische Zusammensetzung gemäß Anspruch 1, wobei besagtes Kollagenmolekül Atelo-Kollagenmolekül umfasst.

3. Pharmazeutische Zusammensetzung gemäß einem der Ansprüche 1 bis 2, wobei besagtes Kollagenmolekül ein fasriges Kollagenmolekül enthält.

4. Pharmazeutische Zusammensetzung gemäß einem der Ansprüche 1 bis 3, wobei besagte Verwendung bei der Behandlung oder Prävention obstruktiver Gefäßerkrankung die Inhibition von Intimahypertrophie umfasst.

5. Pharmazeutische Zusammensetzung gemäß einem der Ansprüche 1 bis 4, wobei besagte Zusammensetzung durch Gel-Bildung besagter Zusammensetzung in der äußeren Schicht der Blutgefäßwand der obstruktiven Gefäßerkrankung verabreicht wird.

6. Pharmazeutische Zusammensetzung gemäß einem der Ansprüche 1 bis 5, wobei besagte Zusammensetzung zur Prävention oder Behandlung von Post-PTCA-Restenose oder Gefäßverschlüssen einschließlich Koronararterie, Gehirngefäß, Nierengefäß und peripherem Gefäß, verwendet wird.

7. Pharmazeutische Zusammensetzung gemäß einem der Ansprüche 1 bis 6, wobei besagtes Nukleinsäure-Konstrukt eine der Sequenzen, wie in SEQ ID NOs: 11 bis 14 dargelegt, targetiert.

8. Medizinische Vorrichtung zur Behandlung oder Prävention obstruktiver Gefäßerkrankung, umfassend:
einen Träger zur Gefäßbehandlung,
pharmazeutische Zusammensetzung gemäß einem der Ansprüche 1 bis 7, welche auf zumindest einem Teil der Oberfläche des Trägers getragen wird, um das Gelieren an der obstruktiven Gefäßerkrankungsstelle zu ermöglichen, wobei besagte pharmazeutische Zusammensetzung an einer Stelle angrenzend zur äußeren Schicht der Blutgefäßwand bereitgestellt wird.

9. Medizinische Vorrichtung gemäß Anspruch 8, wobei besagte medizinische Vorrichtung ein künstliches Blutgefäß oder eine Gefäßprothese ist.

## Revendications

1. Composition pharmaceutique pour utilisation dans la prévention ou le traitement d'une maladie vasculaire obstructive qui résulte d'une resténose après chirurgie vasculaire reconstructive, laquelle composition comprend :
- une construction d'acide nucléique inhibant l'expression du gène de Midkine par interférence d'ARN, laquelle construction d'acide nucléique comprend un petit ARN interférant qui a pour cible au moins une partie de l'ARNm codant la protéine Midkine,
- et une molécule de collagène,
dont le site d'administration est la couche externe du vaisseau sanguin qui est le siège de la maladie vasculaire obstructive.

2. Composition pharmaceutique conforme à la revendication 1, dans laquelle ladite molécule de collagène comprend une molécule d'atélo-collagène.

3. Composition pharmaceutique conforme à la revendication 1 ou 2, dans laquelle ladite molécule de collagène comprend une molécule de collagène fibreux.

4. Composition pharmaceutique conforme à l'une des revendications 1 à 3, dont ladite utilisation dans la prévention ou le traitement d'une maladie vasculaire obstructive comprend l'inhibition d'une hypertrophie de la tunique interne du vaisseau.

5. Composition pharmaceutique conforme à l'une des revendications 1 à 4, laquelle composition est administrée par gélification de ladite composition dans la couche externe de la paroi du vaisseau sanguin qui est le siège de la maladie vasculaire obstructive.

6. Composition pharmaceutique conforme à l'une des revendications 1 à 5, laquelle composition est utilisée pour la prévention ou le traitement d'une resténose postérieure à une angioplastie coronarienne transluminale percutanée ou d'occlusions de vaisseaux, y compris de l'artère coronaire, de vaisseaux du cerveau, de vaisseaux du rein et de vaisseaux périphériques.

7. Composition pharmaceutique conforme à l'une des revendications 1 à 6, dans laquelle ladite construction d'acide nucléique a pour cible n'importe laquelle des séquences données en tant que Séquences N° 11 à 14.

8. Dispositif médical conçu pour le traitement ou la prévention d'une maladie vasculaire obstructive, comportant :
- un support pour traitement de vaisseau,
- et une composition pharmaceutique conforme à l'une des revendications 1 à 7, portée par au moins une partie de la surface du support de manière à pouvoir se transformer en un gel au niveau du siège de la maladie vasculaire obstructive, ladite composition pharmaceutique étant apportée en un site touchant la couche externe de la paroi du vaisseau sanguin.

9. Dispositif médical conforme à la revendication 8, lequel dispositif médical est un vaisseau sanguin artificiel ou une prothèse vasculaire.


### SEQUENCE LISTING

<110> National University Corporation Nagoya University Cell Signals Inc.
<120> Drug Composition for Vascular Occlusive Disorders
<130> FNZZA023 WO
<150> JP2005-152346
   <151> 2005-05-25
<160> 14
<170> PatentIn version 3.3
<210> 1
   <211> 19
   <212> DNA
   <213> Artificial
<220>
   <223> siRNA target sequence of rabbit midkine
<400> 1
   ccaaaaagaa agacaaggt 19
<210> 2
   <211> 19
   <212> DNA
   <213> Artificial
<220>
   <223> siRNA target sequence of rabbit midkine
<400> 2
   ctgaagaagg ctcggtaca 19
<210> 3
   <211> 19
   <212> DNA
   <213> Artificial
<220>
   <223> siRNA target sequence of rabbit midkine
<400> 3
   agaccaaagc caaggccaa 19
<210> 4
   <211> 19
   <212> DNA
   <213> Artificial
<220>
   <223> siRNA target sequence of rabbit midkine
<400> 4
   ccaagaaagg gaaggcaaa 19
<210> 5
   <211> 1.9
   <212> DNA
   <213> Artificial
<220>
   <223> siRNA target sequence of rabbit midkine
<400> 5
   aagggaaggc aaaggacta 19
<210> 6
   <211> 21
   <212> DNA
   <213> Artificial
<220>
   <223> siRNA sense
<400> 6
   ccaaaaagaa agacaaggut t 21
<210> 7
   <211> 21
   <212> DNA
   <213> Artificial
<220>
   <223> siRNA sense
<400> 7
   cugaagaagg cucgguacatt t 21
<210> 8
   <211> 21
   <212> DNA
   <213> Artificial
<220>
   <223> siRNA sense
<400> 8
   agaccaaagc caaggccaat t 21
<210> 9
   <211> 21
   <212> DNA
   <213> Artificial
<220>
   <223> siRNA sense
<400> 9
   ccaagaaagg gaaggcaaatt 21
<210> 10
   <211> 21
   <212> DNA
   <213> Artifcial
<220>
   <223> siRNA sense
<400> 10
   aagggaaggc aaaggacuat t 21
<210> 11
   <211> 19
   <212> RNA
   <213> Artificial
<220>
   <223> siRNA target sequence of human midkine
<400> 11
   gaaggaguuu ggagccgac 19
<210> 12
   <211> 19
   <212> RNA
   <213> Artificial
<220>
   <223> siRNA target sequence of human midkine
<400> 12
   gaaggcgcgc uacaaugcu 19
<210> 13
   <211> 19
   <212> RNA
   <213> Artificial
<220>
   <223> siRNA target sequence of human midkine
<400> 13
   gcaaaggcca aagccaaga 19
<210> 14
   <211> 19
   <212> RNA
   <213> Artificial
<220>
   <223> siRNA target sequence of human midkine
<400> 14
   guuuggagcc gacugcaag 19